(19) Europäisches Patentamt / European Patent Office / Office européen des brevets

(11) **EP 4 281 099 B1**

(12) **EUROPEAN PATENT SPECIFICATION**

(45) Date of publication and mention
of the grant of the patent:
**01.10.2025 Bulletin 2025/40**

(21) Application number: **22743336.4**

(22) Date of filing: **24.01.2022**

(51) International Patent Classification (IPC):
*A61K 9/08* (2006.01)    *A61K 47/02* (2006.01)
*A61K 47/10* (2017.01)    *A61K 47/26* (2006.01)
*A61K 38/00* (2006.01)    *C07K 14/47* (2006.01)
*C07K 14/475* (2006.01)    *C07K 14/81* (2006.01)

(52) Cooperative Patent Classification (CPC):
**A61K 9/08; A61K 47/02; A61K 47/10; A61K 47/26;
C07K 14/4702; C07K 14/475; C07K 14/8121;
A61K 9/0048; A61K 38/00**

(86) International application number:
**PCT/US2022/013547**

(87) International publication number:
**WO 2022/159829 (28.07.2022 Gazette 2022/30)**

(54) **COMPOSITIONS COMPRISING PEDF-DERIVED SHORT PEPTIDES (PDSP) AND USES THEREOF**

ZUSAMMENSETZUNGEN MIT PEDF-ABGELEITETEN KURZEN PEPTIDEN (PDSP) UND VERWENDUNGEN DAVON

COMPOSITIONS COMPRENANT DES PEPTIDES COURTS DÉRIVÉS DE PEDF (PDSP) ET LEURS UTILISATIONS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.01.2021 US 202163140851 P**

(43) Date of publication of application:
**29.11.2023 Bulletin 2023/48**

(73) Proprietor: **Brim Biotechnology, Inc.
Taipei 11492 (TW)**

(72) Inventors:
• **LIAW, Wayne Wei-Cheng
Taipei 11492 (TW)**
• **HUANG, Jason Ping-Yen
Taipei 11492 (TW)**
• **WANG, Emily Hsiao-Han
Taipei 11492 (TW)**
• **LEE, Frank Wen-Chi
Taipei 11492 (TW)**

(74) Representative: **Rommeswinkel, Marike
Von Rohr
Patentanwälte Partnerschaft mbB
Rüttenscheider Straße 62
45130 Essen (DE)**

(56) References cited:
WO-A1-2006/020121    WO-A1-2009/096038
CN-A- 112 206 312    TW-A- 201 412 327
US-A1- 2012 245 097    US-B2- 9 815 878

Remarks:
The complete document including Reference Table(s) and the Sequence Listing(s) can be downloaded from the EPO website

**Description**

BACKGROUND OF INVENTION

Field of Invention

This invention relates to compositions of PEDF-derived short peptides, particularly to formulations of such peptides and uses thereof.

Background

Human Pigment Epithelium-derived Factor (PEDF) is a secreted protein of 418 amino acids, with a molecular weight of about 50 kDa. PEDF is a multifunctional protein with many biological functions (see U.S. Patent Application Publication No. 2010/0047212). Different peptide regions of the human PEDF are found to be responsible for different functions. For example, a 34-mer fragment (residues 44-77 of PEDF) has been identified to have anti-angiogenic activity, while a 44-mer fragment (residues 78-121 of PEDF) has been identified to have neurotrophic properties.

Human PEDF-derived short peptides (PDSPs) have been found to be promising therapeutics for treating or preventing various diseases or disorders. For example. PDSPs are found to be effective in promoting muscle regeneration or arteriogenesis (U.S. Patent No. 9,884,012), treating alopecia and/or hair depigmentation (U.S. Patent No. 9.938,328), treating osteoarthritis (U.S. Patent No 9.777,048), preventing or ameliorating skin aging (U.S. Patent No. 9,815,878), treating liver cirrhosis (U.S Patent No. 8,507,446), or treating various eye diseases or conditions (e g.. retinal degeneration, Meibomian glad disease, dry eye). Corresponding mouse PEDF-derived short peptides (moPDSPs) are also found to have the same therapeutic effects. However, preparations of these peptides were found to lack long-term stabilities. Therefore, there is a need for better formulations for this promising biopharmaceutical product.

SUMMARY OF THE INVENTION

Embodiments of the invention relate to formulations for a PEDF-derived short peptide (PDSP), including SEQ ID NO: 1 (39-mer), SEQ ID NO: 2 (34-mer), SEQ ID NO: 3 (29-mer), SEQ ID NO: 5 (24-mer), SEQ ID NO: 6 (20-mer), SEQ ID NO: 8 (mo29-mer), and SEQ ID NO: 9 (mo20-mer), wherein mo29-mer and mo20-mer are the mouse PDSPs corresponding to the human 29-mer and 20-mer, respectively.

One aspect of the invention relates to an aqueous formulation that includes a PDSP having the sequence of one of SEQ ID NO: 1, 2, 3, 5, 6, 8, or 9; boric acid buffer; and a tonicity agent. The tonicity agent is preferably a non-ionic tonicity agent. The non-ionic tonicity agent may be glycerin, sucrose, mannitol, or sorbitol.

According to some embodiments of the invention, the pH value of the aqueous formulations may be around 5 - 9, preferably around 5.5 - 8.4, more preferably around 6.5-7.5. The tonicity agent is preferably glycerin, which is at a concentration of 10 mM - 500 mM, preferably 200-400 mM, more preferably 300-350 mM. A concentration of the PDSP may be 0.01% - 1% w/v, preferably 0.01-0.1% w/v, more preferably around 0.03%.

Other aspects of the invention would be apparent from the following description and the accompanying drawings.

BRIEF DESCIPTION OF THE DRAWINGS

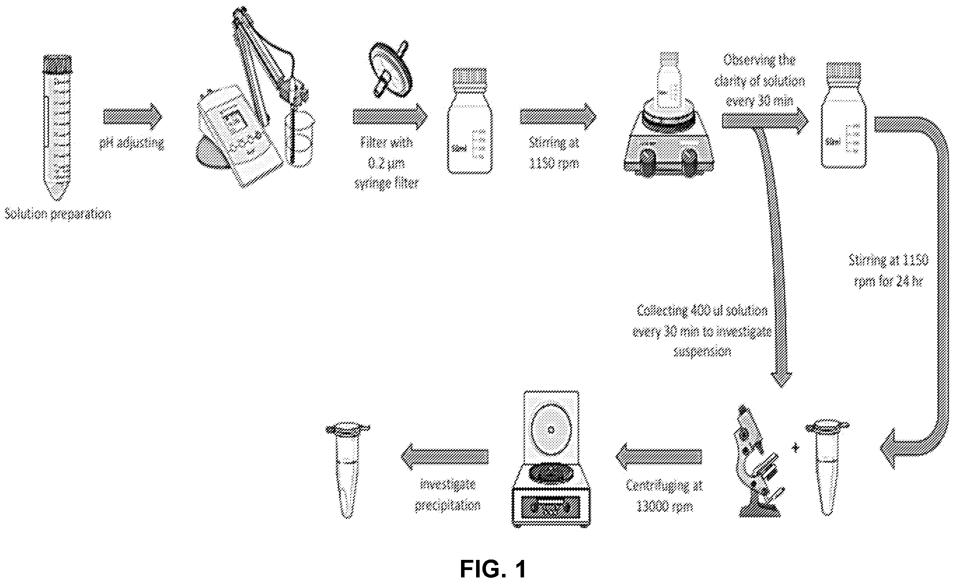

FIG. 1 shows a schematic illustrating a testing protocol for assessing the stabilities of various formulations of PDSP solutions. Different PDSP solutions were prepared according to the study design. The pH values of PDSP solutions were adjusted with 1N HCl or 2N NaOH, filtered through a 0.2 $\mu$m syringe filter, and each placed in a 50 ml glass bottle. The filtered PDSP solutions were stirred at 1,150 RPM at room temperature. Aliquots of 400 $\mu$l PDSP solutions were collected at different time points (every half an hour for the first 7 hours) and centrifuged at 13,000 rpm to observe whether any precipitation had appeared. The stirring of PDSP solutions was continued, and the precipitation was further investigated at 10- and 24-hour time points from the start of stirring. The times when the suspended matter, precipitation, and turbidity appeared were recorded.

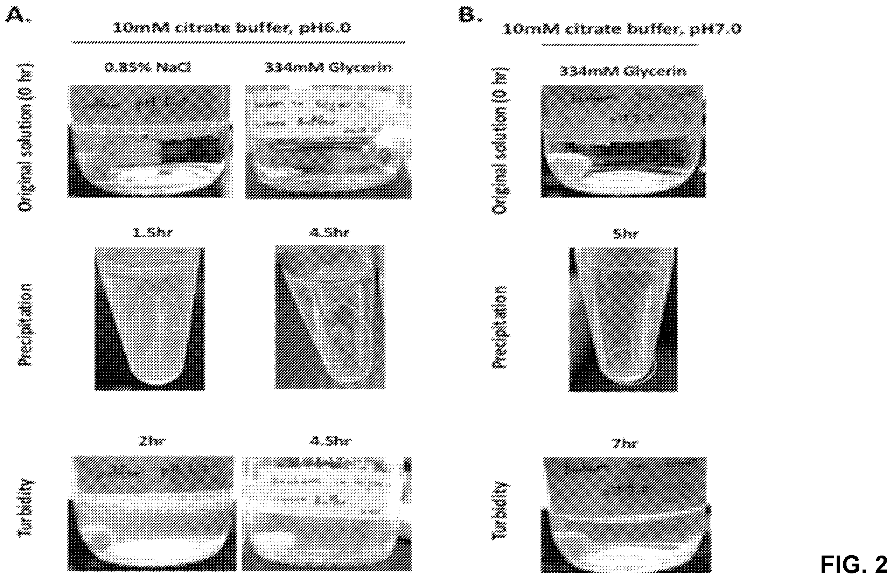

FIG. 2 shows results from stability tests of PDSP formulations prepared in 10 mM citric acid buffer with different excipients and pH values under continuous stirring conditions. (A) PDSP (BRM421) prepared in 10 mM citrate with 0.85% NaCl or 334 mM glycerin, pH 6.0. (B) PDSP (BRM421) prepared in 10mM citrate with 334 mM Glycerin, pH 7.0. These solutions were stirred at 1,150 RPM at room temperature after filtration and investigated every half an hour for the first 7 hours, and also at 10 and 24 hours. The times when precipitation and turbidity showed up were recorded in Table 2.

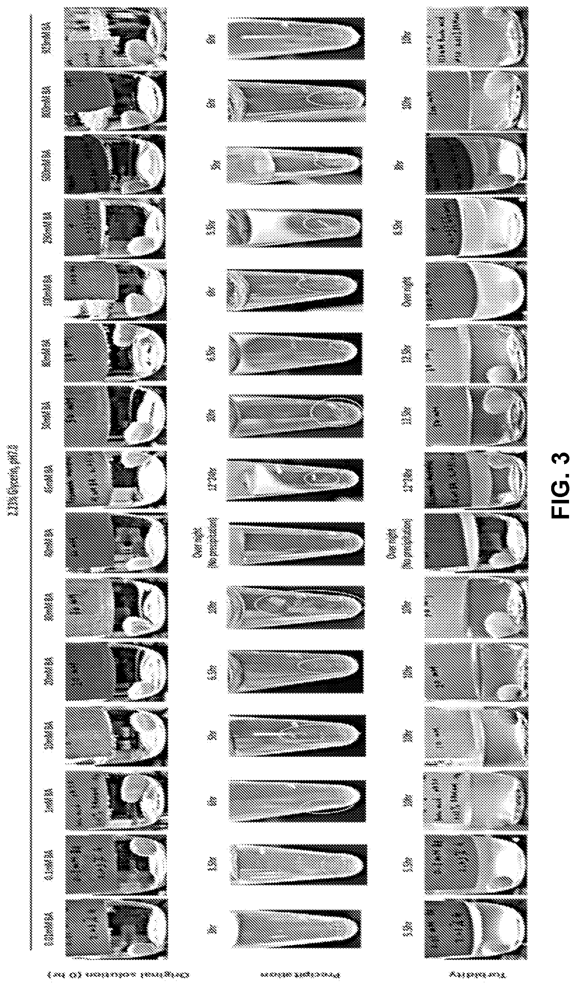

FIG. 3 shows results of stability tests of PDSP (BRM421) formulations prepared with different concentrations of boric acid (BA) and 303 mM glycerin under continuous stirring conditions. PDSP prepared in 15 different formulations were each placed in a 50 mL beaker after filtration, and then stirred at 1,150 RPM at room temperature. These solutions were investigated every half an hour for the first 7 hours, and also at 10, 12.5 and 24 hours. The times when precipitation and turbidity showed up were recorded.

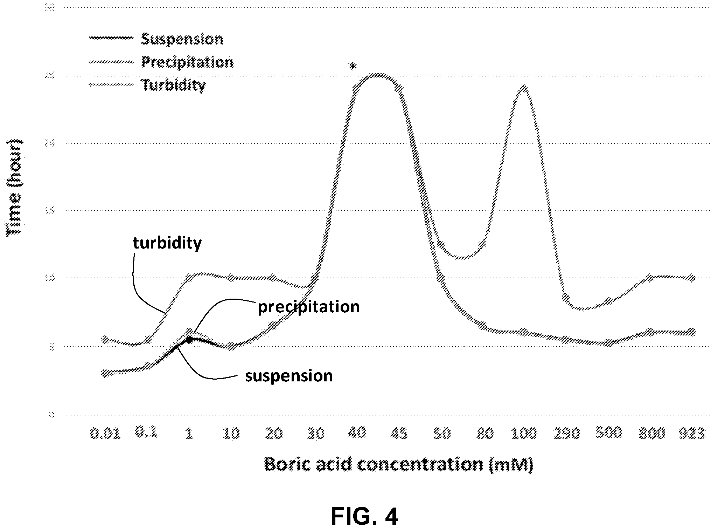

FIG. 4 shows times when suspension, precipitation and turbidity showed up under continuous stirring conditions in PDSP formulations prepared with different concentrations of BA and 303 mM glycerin. Curve 1: Time when suspended matters showed up. Curve 2: Time when visible precipitation showed up. Curve 3: Time when turbid solution showed up. Asterisk: No suspension, precipitation and turbidity were observed in BRM421 prepared in 40 mM BA with 303 mM glycerin within the duration of this study (24 hours). The observation was conducted every half an hour during the 7-hour stirring. And after 7 hours, the observation was conducted at 10, 12.5 and 24 hours. After 7 hours, the time for each phenomenon was recorded when it was first observed.

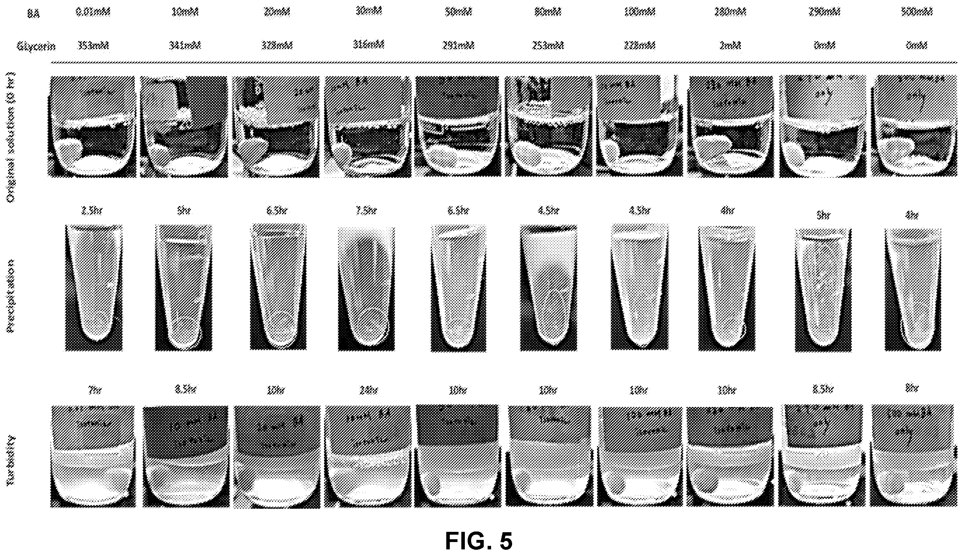

FIG. 5 shows results of stability tests for PDSP solutions prepared in different combinations of BA/glycerin under continuous stirring conditions. PDSP prepared in 8 different concentrations of BA/glycerin solutions and 2 BA only solutions were each placed in a 50 mL beaker after filtration, and then stirred at 1,150 RPM at room temperature. These solutions were investigated every half an hour for the first 10 hours and also at 24 hours.

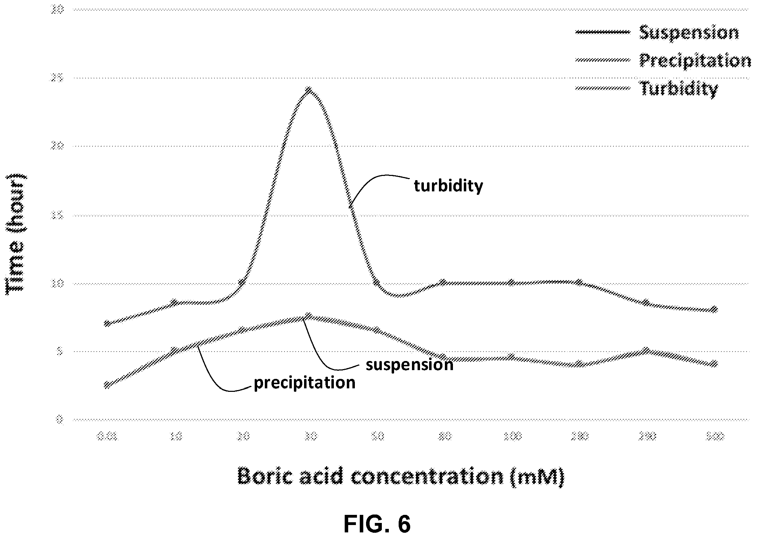

FIG. 6 shows the times when suspension, precipitation, and turbidity showed up under continuous stirring conditions for PDSP solutions prepared in different combinations of BA/glycerin solutions. Curve 1: times when suspended matter showed up. Curve 2: Times when visible precipitation showed up. Curve 3: Times when turbid solution showed up. The observation was conducted every half an hour for the first 10 hours. After 10 hours, the observation was conducted again at 24 hours.

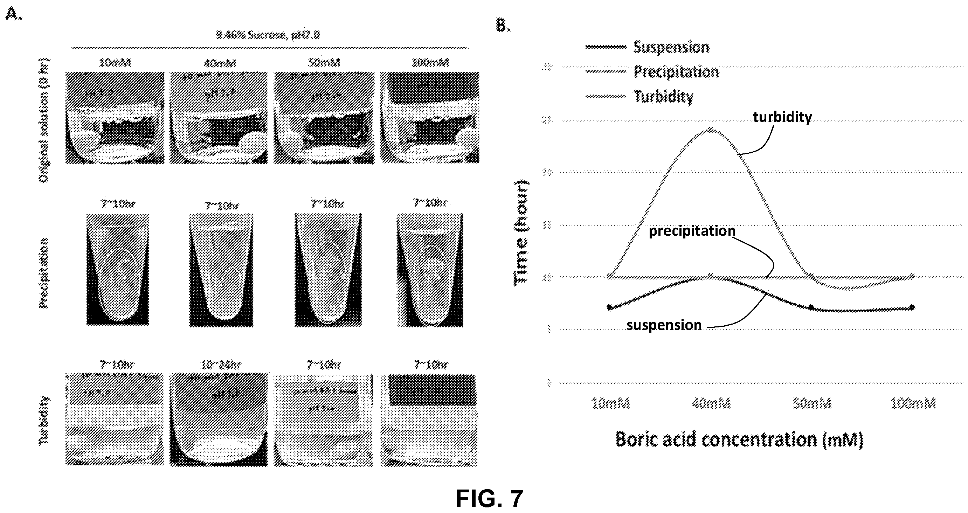

FIG. 7 shows results of stability tests for PDSP solutions prepared in different concentrations of BA with 9.46% sucrose under continuous stirring conditions. **(A)** PDSP (BRM421) prepared in 4 different formulations were each placed in a 50 mL beaker after filtration, and then stirred at 1,150 RPM at room temperature. These solutions were investigated every half an hour for the first 7 hours, and after 10- and 24-hour stirring. **(B)** The line graph of the times when suspension, precipitation, and turbidity showed up under continuous stirring conditions.

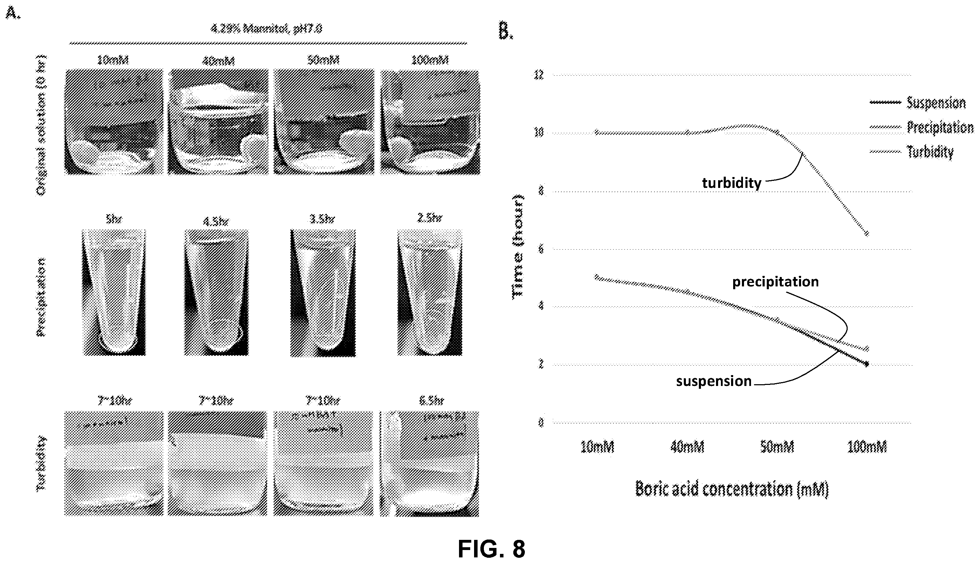

FIG. 8 shows results of stability tests of PDSP (BRM421) prepared in different concentrations of boric acid (BA) with 4.29 % mannitol under continuously stirring condition. **(A)** PDSP prepared in 4 different formulations were each placed in a 50 mL beaker after filtration, and then stirred at 1,150 RPM at room temperature. These solutions were investigated every half an hour for the first 7 hours, and after 10- and 24-hour stirring. **(B)** The chart of the times when suspension, precipitation and turbidity appear under shearing force.

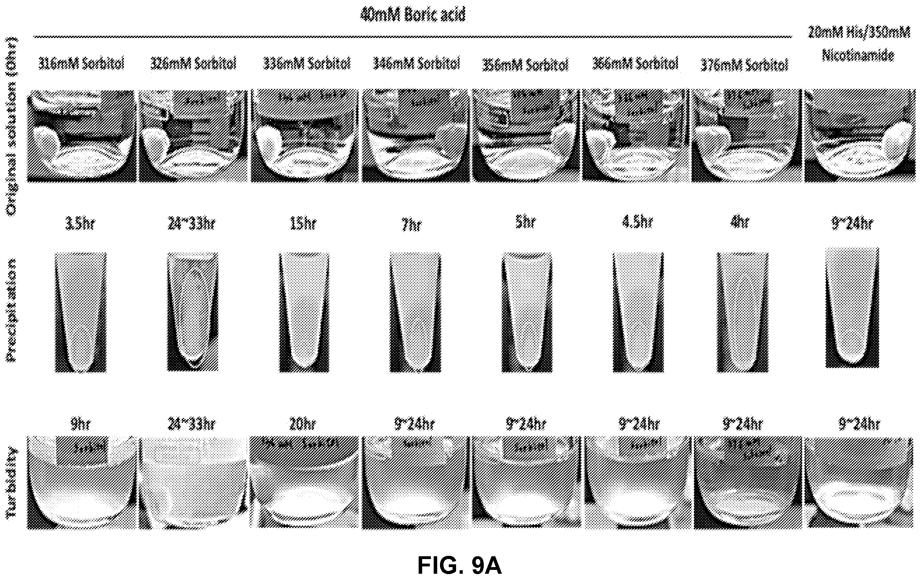

FIG. 9A shows results of stability tests of PDSP (BRM421) in 40 mM boric acid (BA) with various concentrations of sorbitol under shearing force. These solutions were stirred at 1150 RPM at room temperature and investigated every half an hour up to 9 hours. After 9 hours, the BRM421 was continuously stirred and the turbidity was investigated at 24 and 33 hours. The stabilities of BRM421 in 40 mM Boric acid with 326 and 336mM Sorbitol were further investigated from 11 to 20 hours after stirring. The times for suspended matter, precipitation, and turbidity appearance were recorded. The BRM421 in 20 mM Histidine/350 mM Nicotinamide was used as a reference control.

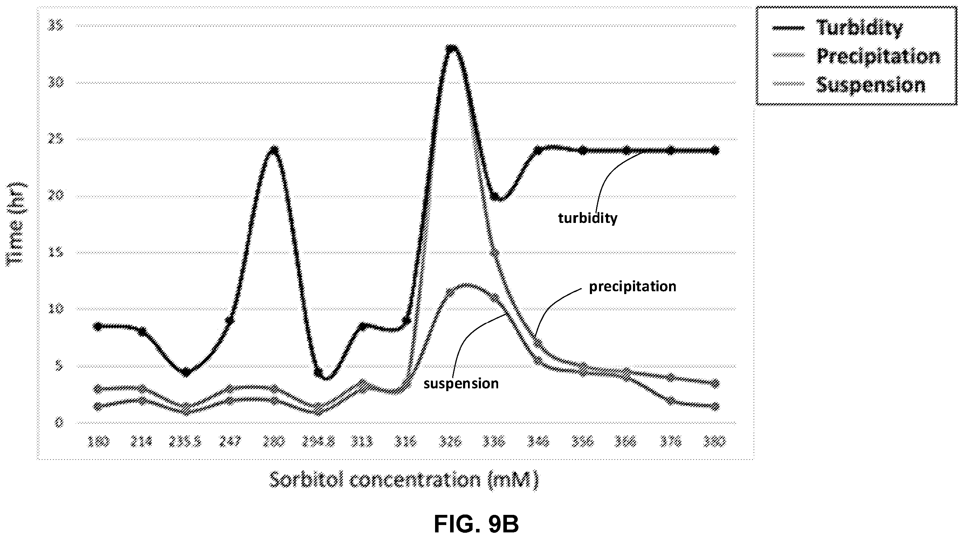

FIG. 9B shows a chart illustrating the times for suspended matter, precipitation, and turbidity appearance from the tests of FIG. 9A.

DETAILED DESCRIPTION

Embodiments of the invention relate to formulations of PEDF-derived short peptides (PDSPs) with enhanced stabilities. Various human PDSPs were found to be promising therapeutics for treating or preventing various diseases or disorders, including muscle regeneration or arteriogenesis, alopecia and/or hair depigmentation, osteoarthritis, skin aging, liver cirrhosis, or eye diseases or conditions. Examples of such PDSPs may include those shown in TABLE 1:

**TABLE 1:** Examples of PEDF derived short peptides (PDSPs)

| Name | Sequence | SEQ ID NO | Human PEDF residues |
|---|---|---|---|
| 39mer | LSVATALSAL**SLGAEQRTESIIBRALYYDL**ISSPDIHGT | 1 | 82-121 |
| 34mer | ALSAL**SLGAEQRTESIIBRALYYDL**ISSPDIHGT | 2 | 88-121 |
| 29mer | **SLGAEQRTESIIHRALYYDL**ISSPDIHGT | 3 | 93-121 |
| 25mer | **EQRTESIIBRALYYDL**ISSPDIHGT | 4 | 97-121 |
| 24mer | **SLGAEQRTESIIHRALYYDL**ISSP | 5 | 93-116 |
| 20mer | **SLGAEQRTESIIHRALYYDL** | 6 | 93-112 |

(continued)

| Name | Sequence | SEQ ID NO | Human PEDF residues |
|------|----------|-----------|---------------------|
| 18mer | **EQRTESIIBRALYYDL**IS | 7 | 97-114 |
| mo29mer | **SLGAEHRTESVIHRALYYDL**ITNPDIHST | 8 | mouse |
| mo20mer | **SLGAEHRTESVIHRALYYDL** | 9 | mouse |

In accordance with embodiments of the invention, the PDSPs may be SEQ ID NO: 1, 2, 3, 5, 6, 8, or 9. In addition, the N-termini of these peptides may be optionally protected with acylation (e.g., acetyl or propionyl), and the C-termini may be optionally protected as amides.

These PDSPs have been prepared in citrate buffers and found to be effective for therapeutic purposes in various pre-clinical studies. However, preparations of these short peptides (e.g., BRM421, 29mer PDSP (SEQ ID NO:3) in 10 mM citrate buffer with 0.85% w/v NaCl, pH 6.0) were found to lack long-term stabilities (over several months).

Many factors, including chemical stress (e.g., oxidation, hydrolysis, etc.) and physical stress (e.g., temperature, light, and agitation), can affect the qualities and stabilities of biopharmaceutical products, particularly during long-term storage. To investigate the stabilities of PDSP in different formulations, accelerated stability testing was performed. Specifically, various formulations were tested under stress conditions, particularly under shear stress, to identify optimal formulations. After extensive tests, formulations in boric acid buffers are unexpectedly found to have long-term stabilities remarkably superior to those of the original citrate buffer formulations.

Boric acid has mild antibiotic properties that can be used to treat fungal or bacterial infections. Boric acid has been used in ophthalmic solutions as eye wash to cleanse or irrigate the eyes. Boric acid can also provide soothing relief from eye irritations. In addition, boric acid also functions as a buffering agent and/or tonicity-adjusting agent in the ophthalmic solutions. Embodiments of the invention take advantage of these beneficial effects of boric acid, as well as its unexpected ability to promote long-term stabilities of PDSP formulations.

The following describes specific examples to illustrate embodiments of the invention. However, one skilled in the art would appreciate that these specific examples are for illustration only. For example, even though the following examples use BRM421 DP (29mer PDSP; SEQ ID NO:3) for illustrations, other PDSPs may be used instead.

1. Citrate Buffer (10 mM working Citrate Buffer with 0.85% w/v NaCl, pH6.0)

Citrate buffers were prepared from citrate acid and trisodium citrate to achieve the desired buffer capacity and pH values. For example, citrate acid monohydrate (MW 210.14 Da) (Merck) and trisodium citrate dihydrate (MW 294.12 Da) (BioShop) were used to prepare solution A and solution B, respectively. These two solutions are then used to make the citrate buffers with the desired concentrations and pH values. The formula of solutions A and B, for example, are as follows:

Solution A (0.1 M citrate acid monohydrate) (10 ml): 210.14 Da $\times$ 10/1000 $\times$ 0.1 = 0.21 g citrate acid monohydrate. Weigh 0.21 g Citrate acid monohydrate and dissolve it in 10 ml ddH$_2$O to produce a 10 ml solution A stock.

Solution B (0.1 M trisodium citrate dihydrate) (10 ml): 294.12 Da $\times$ 10/1000 $\times$ 0.1 = 0.294g trisodium citrate dihydrate. Weigh 0.294 g trisodium citrate dihydrate and dissolve it in 10 ml ddH$_2$O to produce a 10 ml solution B stock.

As an example, to prepare a 10X citrate buffer stock pH 6.0, 1.15 ml solution A and 8.85 ml solution B were mixed to obtain a 0.1 M citrate buffer, 10mL. Then, the 10 ml, 0.1 M citrate buffer stock was diluted with 90 ml ddH$_2$O to generate a 10 mM working citrate buffer, 100 ml (1X solution). To prepare a 10 mM citrate buffer with 0.85% w/v NaCl, 0.85 g NaCl was added into the 10 mM working citrate buffer, 100 ml. Before use, pH should be measured and adjusted based on study design.

2. Boric acid buffer (1-923 mM boric acid buffers with 0.77% w/v NaCl or 303 mM glycerin or 9.46% w/v sucrose or 4.29% w/v mannitol, pH 7.0)

Formulations of the invention are prepared in various boric acid buffers. To prepare 20 mL boric acid buffers of various concentrations at pH 7.0 for testing, the following formula may be used to calculate the amount of boric acid needed: 61.83 (Da) $\times$ 0.000001 $\times$ X (mM) $\times$ Y (final solution mL) = BA (g). Specifically, the following boric acid buffers were prepared in the studies: 0.01mM, 0.1mM, 1mM, 10mM, 20mM, 30mM, 40mM, 45mM, 50mM, 80mM, 100mM, 280mM, 290mM, 500mM, 800mM, and 923mM (923 mM is the saturated concentration of BA in 25 °C water).

Various tonicity adjusting agents have been used in ophthalmic formulations, such as NaCl, sucrose, glycerin, mannitol, and sorbitol. For example, to prepare 20 mL boric acid (BA) solutions pH 7.0 with such tonicity agents, different amounts of BA were dissolved in 15 mL ddH$_2$O, and then 512.6 μl 87% Glycerin, or 0.154 g NaCl, or 1.892 g Sucrose, or 0.858 g Mannitol, was added into the BA solutions to prepare the boric acid buffers with 303 mM Glycerin, 0.77% NaCl, 9.46% sucrose, 4.29% mannitol, respectively. The pH values of the buffers were adjusted to pH 7.0 using 2N NaOH. The volumes of 2N NaOH for pH value adjustments were recorded, and then ddH$_2$O was added to a total of 20 ml.

3. Preparation of BA/glycerin buffers with different concentrations of boric acid (0.01-280 mM) and glycerin at pH7.0

Glycerin is a tonicity agent and is also used as a lubricant in ophthalmic formulations. Therefore, different concentrations of glycerin in boric acid buffers were prepared and investigated for the stability of BR421 DP formulations. In these boric acid buffers, the amounts of glycerin were adjusted to maintain substantially the same tonicity as follows. The concentration (%) of glycerin needed for X mM boric acid buffer can be calculated using the following formula:

$$\{100 - [(X \ (mM) \div 40) \times 14.2]\} \div 100 \times 2.6 = \text{glycerin} \ \%$$

For example: (the glycerin used in this study is a 87% solution)

0.01 mM BA/353 mM glycerin:

$$\{100-[(0.01\div40)\times14.2]\}\div100\times2.6=2.5999077 \ \%$$

Glycerin=597.68 μl/20ml (i.e., 597.68 μl of 87% glycerin solution is needed to achieve 2.5999077 % glycerin in 20 ml total volume)

10mM BA/341mM glycerin:

$$\{100-[(10\div40)\times14.2]\}\div100\times2.6=2.5077 \ \%$$

Glycerin=576.48 μl/20ml (i.e., 576.48 μl of 87% glycerin solution is needed to achieve 2.5077 % glycerin in 20 ml total volume)

20mM BA/328mM glycerin:

$$\{100-[(20\div40)\times14.2]\}\div100\times2.6=2.4154 \ \%$$

Glycerin=555.26 μl/20ml (i.e., 555.26 μl of 87% glycerin solution is needed to achieve 2.4154 % glycerin in 20 ml total volume)

30mM BA/316mM glycerin:

$$\{100-[(30\div40)\times14.2]\}\div100\times2.6=2.3231 \ \%$$

Glycerin=534.05 μl/20ml (i.e., 534.05 μl of 87% glycerin solution is needed to achieve 2.3231 % glycerin in 20 ml total volume)

50mM BA/291mM glycerin:

$$\{100-[(50\div40)\times14.2]\}\div100\times2.6=2.1385 \ \%$$

Glycerin=491.61 μl/20ml (i.e., 491.61 μl of 87% glycerin solution is needed to achieve 2.1385 % glycerin in 20 ml total volume)

80mM BA/253mM glycerin:

$$\{100-[(80\div40)\times14.2]\}\div100\times2.6=1.8616 \ \%$$

Glycerin=427.95 μl/20ml (i.e., 427.95 μl of 87% glycerin solution is needed to achieve 1.8616 % glycerin in 20 ml total volume)

100mM BA/228mM glycerin:

$$\{100-[(100\div40)\times14.2]\}\div100\times2.6=1.677\ \%$$

Glycerin=385.52 µl/20ml (i.e., 385.52 µl of 87% glycerin solution is needed to achieve 1.677 % glycerin in 20 ml total volume)

280mM BA/2mM glycerin:

$$\{100-[(280\div40)\times14.2]\}\div100\times2.6=0.0156\ \%\ \text{Glycerin}=$$

3.59 µl/20ml (i.e., 3.59 µl of 87% glycerin solution is needed to achieve 0.0156 % glycerin in 20 ml total volume)

To prepare 20 mL BA buffer solutions pH 7.0, different amounts of BA (as described above) and different amounts of glycerin were dissolved in 15 mL ddH$_2$O as described above. The pH values of this buffers were adjusted to pH 7.0 using 2N NaOH. The volumes of 2N NaOH for pH value adjustments were recorded, and then ddH$_2$O was added to a total of 20 ml.

4. Preparation of PDSP (BM421 DP) in different formulations

The PDSP used in these examples is BRM421, which is a short synthetic peptide (29mer; SEQ ID NO:3) with acetylation at the N terminus and an amide at the C terminus. Other PDSPs can be similarly prepared. The BM421 peptide was synthesized and characterized by Bachem (Lot:7003945, peptide content: 88.6%; Torrance, CA). The molecular weight of BRM421 is 3243.6 Da. As an example, BRM421 concentration in BRM421 DP solutions for the following studies is 0.03% w/v in the buffers/solutions described above. The calculation formula for a 20 mL solution is as follow:

$$0.03\%\ \text{BRM421} = 0.03\text{g}/100\text{ml} = 0.0003\text{g}/\text{ml}$$

$$0.0003\ \text{g} \div 88.6\%\ \text{peptide content} = 0.0003386\ \text{g}$$

$$0.0003386 \times 20 = 0.006772\ \text{g}$$

e.g., 20 ml BA buffer or citrate buffer + 0.006772 g Bachem BRM421 = 20 ml, 0.03% BRM421 DP
The pH value of BRM421 DP was measured after BRM421 completely dissolved in the solution and then adjusted to 7.0 or 6.0 according to the study designs. Before use in the study, BRM421 DP should be filtered with a 0.2µm syringe filter. PDSPs at different concentrations can be similarly prepared.

5. Evaluation of BRM421 DP prepared in different formulations for their abilities to resist shearing force

We noticed that earlier formulations of PDSP in citrate buffers were not stable during long-term storage (over several months). To test the effects of different formulations on the stability, the various PDSP formulations were subject to stress conditions (e.g., shear stress) to accelerate the changes.
For these tests, twenty (20) milliliters of PDSP (BRM421) prepared in different buffers and excipients (as shown in **Table 2)** were each placed in a 50 mL beaker after filtration. Then, the solutions were subject to stirring at 1,150 RPM at room temperature and observed. Aliquots of 400 µl PDSP solutions each were collected into 1.5 ml Eppendorf tubes every half an hour up to 7 hours. The collected samples were centrifuged at 13,000 rpm for 5 min to evaluate whether any precipitation had occurred. After the continuous observation, the stirring of PDSP solution was continued up to 24 hours to investigate precipitations. The solution appearance and possible precipitation were investigated after 10-hour and 24-hour stirring. The times when suspended matter, precipitation, and turbidity appeared were recorded. The experimental procedures are illustrated in **FIG. 1.**

**Table 2. List of the formulations tested in this study.**

| Excipients | Base buffer | pH value |
| --- | --- | --- |
| 334mM Glycerin | 10mM citrate buffer | 6.0 |
| 334mM Glycerin | 10mM citrate buffer | 7.0 |

(continued)

| Excipients | Base buffer | pH value |
|---|---|---|
| 0.85% NaCl | 10mM citrate buffer | 6.0 |
| 303mM Glycerin | 0.01mM BA buffer | 7.0 |
| 303mM Glycerin | 0.1mM BA buffer | 7.0 |
| 303mM Glycerin | 1mM BA buffer | 7.0 |
| 303mM Glycerin | 10mM BA buffer | 7.0 |
| 303mM Glycerin | 20mM BA buffer | 7.0 |
| 303mM Glycerin | 30mM BA buffer | 7.0 |
| 303mM Glycerin | 40mM BA buffer | 7.0 |
| 303mM Glycerin | 45mM BA buffer | 7.0 |
| 303mM Glycerin | 50mM BA buffer | 7.0 |
| 303mM Glycerin | 80mM BA buffer | 7.0 |
| 303mM Glycerin | 100mM BA buffer | 7.0 |
| 303mM Glycerin | 290mM BA buffer | 7.0 |
| 303mM Glycerin | 500mM BA buffer | 7.0 |
| 303mM Glycerin | 800mM BA buffer | 7.0 |
| 303mM Glycerin | 923mM BA buffer | 7.0 |
| 353mM Glycerin | 0.01mM BA buffer | 7.0 |
| 341mM Glycerin | 10mM BA buffer | 7.0 |
| 328mM Glycerin | 20mM BA buffer | 7.0 |
| 316mM Glycerin | 30mM BA buffer | 7.0 |
| 291mM Glycerin | 50mM BA buffer | 7.0 |
| 253mM Glycerin | 80mM BA buffer | 7.0 |
| 228mM Glycerin | 100mM BA buffer | 7.0 |
| 2mM Glycerin | 280mM BA buffer | 7.0 |
| 0mM Glycerin | 290mM BA buffer | 7.0 |
| 0mM Glycerin | 500mM BA buffer | 7.0 |
| 0.77% NaCl | 40mM BA buffer | 7.0 |
| 9.46% Sucrose | 10mM BA buffer | 7.0 |
| 9.46% Sucrose | 40mM BA buffer | 7.0 |
| 9.46% Sucrose | 50mM BA buffer | 7.0 |
| 9.46% Sucrose | 100mM BA buffer | 7.0 |
| 4.29% Mannitol | 10mM BA buffer | 7.0 |
| 4.29% Mannitol | 40mM BA buffer | 7.0 |
| 4.29% Mannitol | 50mM BA buffer | 7.0 |
| 4.29% Mannitol | 100mM BA buffer | 7.0 |

Results

**1.** The ability to resist shearing force for BRM421 PD prepared in 10 mM citrate buffer with 0.85% w/v NaCl, pH 6.0

The original formulation for BRM421 DP is 10 mM citrate buffer with 0.85% w/v NaCl, pH 6.0. This formulation was fine for various pre-clinical studies. However, this formulation developed turbidity over a long-term storage (many months). Therefore, its stability was investigated using forced aggregation method to elucidate its ability to resist shearing force. As shown in **FIG. 2,** solution was clear and transparent before stirring **(FIG. 2,** upper panel). The suspended matter was seen in this formulation around 1 hour after the start of stirring **(FIG. 2,** left panel and **Table 3).** The precipitation and turbid solution were observed 1.5 and 2.5 hours after start of the stirring, respectively. These observations will be used as baseline for comparison with other formulations.

**Table 3. The stabilities of** BRM421 DP **prepared in different formulations under stirring conditions**

| Solution | | Suspension (hour) | Precipitation (hour) | Turbidity (hour) |
|---|---|---|---|---|
| Excipients/pH | Base buffer | | | |
| 0.85% NaCl, pH 6.0 | 10 mM citrate buffer | 1 | 1 | 2 |
| 334 mM Glycerin, pH 6.0 | 10 mM citrate buffer | 4.5 | 4.5 | 4.5 |
| 334 mM Glycerin, pH 7.0 | 10 mM citrate buffer[1st] | 5 | 5 | 7 |
| | 10 mM citrate buffer[2nd] | 4.5 | 5 | 7 |
| 303 mM Glycerin, pH 7.0 | 0.01 mM BA buffer | 3 | 3 | 5.5 |
| | 0.1 mM BA buffer | 3.5 | 3.5 | 5.5 |
| | 1 mM BA buffer | 5.5 | 6 | Between 7-10 |
| | 10 mM BA buffer | 5 | 5 | Between 7-10 |
| | 20 mM BA buffer | 6.5 | 6.5 | Between 7-10 |
| | 30 mM BA buffer | Between 7-10 | Between 7-10 | Between 7-10 |
| | 40 mM BA buffer@ | No suspension | No precipitation | No turbidity |
| | 45 mM BA buffer[1st] | Between 12-24 | Between 12-24 | Between 12-24 |
| | 45 mM BA buffer[2nd] | Between 10-24 | Between 10-24 | Between 10-24 |
| | 50 mM BA buffer | Between 7-10 | Between 7-10 | Between 10-12.5 |
| | 80 mM BA buffer | 6.5 | 6.5 | Between 10-12.5 |
| | 100 mM BA buffer@ | 6 | 6 | Between 10-24 |
| | 290 mM BA buffer | 5.5 | 5.5 | 8.5 |
| | 500 mM BA buffer[1st] | 5.5 | 5.5 | 8.5 |
| | 500 mM BA buffer[2nd] | 5 | 5 | 8 |
| | 500 mM BA buffer[3rd] | 4.5 | 5 | Between 7-10 |
| | 800 mM BA buffer | 6 | 6 | Between 10-24 |
| | 923 mM BA buffer | 6 | 6 | Between 7-10 |
| 353 mM Glycerin, pH 7.0 | 0.01 mM BA buffer | 2.5 | 2.5 | 7 |
| 341 mM Glycerin, pH 7.0 | 10 mM BA buffer | 5 | 5 | 8.5 |
| 328 mM Glycerin, pH 7.0 | 20 mM BA buffer | 6.5 | 6.5 | 10 |
| 316 mM Glycerin, pH 7.0 | 30 mM BA buffer | 7.5 | 7.5 | Between 10-24 |
| 291 mM Glycerin, pH 7.0 | 50 mM BA buffer | 6.5 | 6.5 | 10 |
| 253 mM Glycerin, pH 7.0 | 80 mM BA buffer | 4.5 | 4.5 | 10 |
| 228 mM Glycerin, pH 7.0 | 100 mM BA buffer | 4.5 | 4.5 | 10 |
| 2 mM Glycerin, pH 7.0 | 280 mM BA buffer | 4 | 4 | 10 |

(continued)

| Solution | | Suspension (hour) | Precipitation (hour) | Turbidity (hour) |
|---|---|---|---|---|
| Excipients/pH | Base buffer | | | |
| 0 mM Glycerin, pH 7.0 | 290 mM BA buffer | 5 | 5 | 8.5 |
| 0 mM Glycerin, pH 7.0 | 500 mM BA buffer | 4 | 4 | 8 |
| 9.46% Sucrose, pH 7.0 | 10 mM BA buffer | 7 | Between 7-10 | Between 7-10 |
| | 40 mM BA buffer | Between 7-10 | Between 7-10 | Between 10-24 |
| | 50 mM BA buffer | 7 | Between 7-10 | Between 7-10 |
| | 100 mM BA buffer | 7 | Between 7-10 | Between 7-10 |
| 4.29% Mannitol, pH 7.0 | 10 mM BA buffer[1st] | 5 | 5 | 7-10 |
| | 10 mM BA buffer[2nd] | 4.5 | 4.5 | 7-10 |
| | 40 mM BA buffer[1st] | 4.5 | 4.5 | 7-10 |
| | 40 mM BA buffer[2nd] | 4.5 | 4.5 | 7-10 |
| | 50 mM BA buffer[1st] | 3.5 | 3.5 | 7-10 |
| | 50 mM BA buffer[2nd] | 3.5 | 4 | 7 |
| | 100 mM BA buffer[1st] | 2 | 2.5 | 6.5 |
| | 100 mM BA buffer[2nd] | 2.5 | 3 | 6 |
| 0.77% NaCl, pH 7.0 | 40 mM BA buffer | 5 | 5 | 5.5 |
| 316 mM Sorbitol, pH 7.0 | 40 mM BA buffer | 4 | 4 | 9 |
| 326 mM Sorbitol, pH 7.0 | 40 mM BA buffer | 12 | 33 | 33 |
| 336 mM Sorbitol, pH 7.0 | 40 mM BA buffer | 11 | 15 | 20 |

@ These 2 sets of study had been conducted twice to confirm the results and the same results from both studies were obtained.

**2.** The abilities to resist shearing forces for BRM421 DP prepared in BA-based buffers and citrate-based buffers.

To assess the stability of BRM421 DP prepared in BA buffer and citrate buffer under stirring conditions, 10 mM citrate buffer with 334 mM glycerin, pH 6.0 or pH 7.0 and 40 mM BA buffer with 303 mM glycerin, pH 7.0 were chosen for comparison. The difference in glycerin concentrations between these two formulations is due to different osmolarities between the BA and citrate buffers - i.e., different amounts of glycerin are needed to keep tonicity the same. The suspended matter and precipitation were seen in BRM421 DP prepared in citrate-based buffer within 4.5 to 5 hours after stirring no matter what pH values were **(FIG. 2B** and **Table 3)**. However, neither suspended matter nor precipitation was observed even after 24-hour stirring of BRM421 DP prepared in BA-based buffer **(FIG. 3** and **Table 3)**. These data indicate that BA is a more suitable base buffer for BRM421 DP.

**3.** The ability to resist shearing force for BRM421 DP prepared in 303 mM Glycerin with different concentrations of BA solutions

To investigate which concentration of BA is better for stability, BRM421 DP formulations prepared in 15 different concentrations of BA (0.01-923mM) with 303 mM Glycerin were tested **(Table 3)**. Suspended matters were observed after 3, 3.5, 5.5, 5, 6.5, 7-10, >24, 12-24, 7-10, 6.5, 6, 5.5, 5, 6, and 6 hours after stirring for BRM421 DP formulations prepared in 0.01-, 0.1-, 1-, 10-, 20-, 30-, 40, 45, 50-, 80-, 100-, 290-, 500-, 800-, and 923-mM BA/303 mM glycerin, respectively. **(FIGs. 3, 4** and **Table 3)**.

In our previous study, we found that the suspended matter in BRM421 DP formulations prepared in 10 mM citrate buffer/0.85% NaCl was coarse and could be seen under the dissection microscope as small particle or fiber. However, the suspended matter in the BRM421 DP formulations prepared in BA buffers were fine, which only decreased the solution transparency without visible particle even under the dissection microscope.

To evaluate whether precipitations were also observed when suspended matter appeared, 400 μl of each BRM421 DP formulations was collected into a 1.5ml Eppendorf tube for centrifugation (13,000 rpm for 5min). As shown in **FIG. 3,** visible precipitations were observed at 3, 3.5, 6, 5, 6.5, 7-10, >24, 12-24, 7-10, 6.5, 6, 5.5, 5, 6, and 6 hours after stirring for BRM421 DP formulations prepared in 0.01-, 0.1-, 1-, 10-, 20-, 30-, 40-, 45-, 50-, 80-, 100-, 290-, 500-, 800-, and 923-mM BA/303 mM glycerin, respectively. **(FIG. 3,** middle panel, **FIG. 4,** and **Table 3).**

BRM421 DP solutions were continuously stirred until solution turned turbid after suspended matters or precipitations were observed. **FIG. 3** showed that BRM421 DP formulations prepared in 0.01-, 0.1-, 1-, 10-, 20-, 30-, 40-, 45-, 50-, 80-, 100-, 290-, 500-, 800-, and 923-mM BA/303 mM glycerin became turbid at 5.5, 5.5, 7-10, 7-10, 7-10, 7-10, >24, 12-24, 10-12.5, 10-12.5, 10-24, 8.5, 8.5, 10-24, and 7-10 hours after stirring, respectively. **(FIG. 3,** bottom panel, **FIG. 4,** and **Table 3).**

Among these formulations, BRM421 DP formulation prepared in 40 mM BA/303 mM glycerin was the only one remained clear after 24-hour stirring. **(FIGs. 3, 4** and **Table 3).**

All these results together suggest that there is an optimal BA concentration range around 30-100 mM in the presence of about 303 mM glycerin that can confer the most stability to the BRM421 DP formulations.

**4. The ability to resist shearing force for BRM421 DP prepared by different combinations of Glycerin/BA solution**

To assess whether the osmolality affects the ability to resist shearing force, BRM421 DP formulations prepared by 8 different combinations of BA/Glycerin and 2 BA only buffers were investigated **(Table 2).** As shown in Figure 5, all solutions were clear and transparent before stirring **(FIG. 5,** upper panel). Suspended matters and precipitations were observed at 2.5, 5, 6.5, 7.5, 6.5, 4.5, 4.5 and 4 hours after stirring for BRM421 DP solutions prepared in 0.01/353-, 10/341-, 20/328-, 30/316-, 50/291-, 80/253-, 100/228- and 280/2-mM BA/glycerin buffers, respectively. For BRM421 DP prepared in 290- and 500-mM BA only buffers, suspended matters and precipitations were observed at 5 and 4 hours after stirring, respectively. **(FIGs. 5, 6** and **Table 3).**

The solutions turned turbid at 7, 8.5, 10, 24, 10, 10, 10 and 10 hours after stirring in BRM421 DP formulations prepared in 0.01/353-, 10/341-, 20/-328, 30/316-, 50/291-, 80/253-, 100/228- and 280/2-mM BA/glycerin buffers, respectively. For BRM421 DP formulations prepared in 290- and 500- mM BA only buffers, solution became turbid at 8.5 and 8 hours after stirring, respectively **(FIG. 5,** bottom panel, **FIG. 6** and **Table 3).** These results show that the buffer compositions around 30 mM BA and 316 mM glycerin provide the best stabilities.

**5. The ability to resist shearing force for BRM421 DP formulations prepared in BA buffers with different excipients**

To assess whether other excipients (other than glycerin) could also work with BA buffer to improve the stabilities of the formulations, BRM421 DP formulations were prepared in 40mM BA-based buffers with NaCl, sucrose, mannitol, or sorbitol as the excipients.

As shown in **FIG. 7** and **Table 3,** the suspended matters were found at 7, 10,7 and 7 hours after stirring in BRM421 DP formulations prepared in 10-, 40-, 50- and 100-mM BA buffers with 9.46% sucrose, respectively. And for BRM421 DP formulations prepared in 10-, 40-, 50- and 100-mM BA buffer with 4.29% mannitol, the suspension matters were found at 5, 4.5, 3.5 and 2 hours after stirring, respectively **(FIG. 8** and **Table 3).** The time for suspension matter appeared in both of BA/sucrose and BA/mannitol are quicker than in 40mM BA/303mM glycerin, suggesting that these excipients are not as good for stabilization of the BRM421 DP formulations.

As shown in FIGs. 9A and 9B and Table 3, for BRM421 DP formulations prepared in 40 mM BA and sorbitol (180 mM - 380 mM), the most stable formulations were found with sorbitol concentrations around 316 - 346 mM, particularly with about 326 mM sorbitol. For example, with 326 mM sorbitol and 40 mM BA, suspended matter was seen in solutions around 12 hours after stirring, precipitation was observed around 33 hours after stirring, and turbidity was observed around 33 hours after stirring.

These results together suggest that the non-ionic tonicity agents are better for BRM421 in boric acid buffers than ionic tonicity agents, and that sorbitol and glycerin are more suitable excipients for BRM421 DP than sucrose or mannitol.

The above examples clearly show that formulations of PDSP in boric acid-based buffers with sorbitol, glycerin or other tonicity agents can afford dramatically improved stabilities, as compared to the original formulations in citrate-based buffers. For example, precipitations appeared within 1 hour of stirring of BRM421 DP formulations prepared in 10 mM citrate/0.85% NaCl, while precipitation was observed after 5-hour stirring for BRM421 DP formulations prepared in 40 mM BA with 0.77% NaCl.

When NaCl (tonicity agent) was replaced with non-ionic tonicity agents (e.g., sorbitol or glycerin), the stabilities of the formulations are greatly improved. For example, the time for precipitation appearing was similar for BRM421 DP formulations prepared in 10 mM citrate/334 mM glycerin (4.5 hours) and BRM421 DP formulations prepared in 0.01 mM BA/303 mM glycerin (3 hours), where 0.01 mM BA possess extremely low buffer capacity. For BRM421 DP formulations prepared in 40 mM BA/303 mM glycerin, neither suspended matter nor precipitation was observed even after 24-hour stirring at room temperature. These data indicated that BA is a remarkably better choice for BRM421 DP formulations than citrate as base buffer.

To further assess whether different concentrations of tonicity agents (e.g., glycerin) could affect stabilities of BRM421 DP formulations, different combinations of BA and glycerin were chosen to achieve same osmolality, and the stabilities of BRM421 DP formulations in these buffers were tested by stirring. Among all combinations of BA and glycerin, the formulations with 20 - 100 mM BA and 228 - 328 mM glycerin produced more stable combinations. In particular, formulations of BA with 303 mM glycerin showed the best stability based on the time when precipitation appeared, while other combinations have precipitation appeared sooner or at about the same time. This observation further confirms that around 303 mM glycerin is a better choice for BRM421 DP formulations prepared in BA buffers. Similarly, it was found that with 40 mM BA and 316 - 336 mM sorbitol more stable formulations are obtained. In particular, with around 326 mM sorbitol and 40 mM BA, a very stable formulation is obtained.

In addition to the base buffers, different types and concentrations of excipients other than glycerin were also investigated for their abilities to promote stability of BRM421 DP formulations. Among the 38 formulations investigated, the average time for suspended matters to show up in 9.46% sucrose buffers was at least 7 hours, which is better than those of mannitol and NaCl but worse than that of glycerin. Compared to the results of 40 mM BA/0.77% w/v NaCl, non-ionic tonicity agents, such as glycerin, sorbitol, sucrose, and mannitol, performed better than the ionic tonicity agent. Based on these results, embodiments of the invention preferably use non-ionic tonicity agents, e.g., glycerin, sucrose, sorbitol, and mannitol.

Embodiments of the invention have been illustrated with a limited number of examples. One skilled in the art would appreciate that these examples are for illustration only and are not meant to limit the scope of the invention.

Accordingly, the scope of the invention should be limited by the attached claims.

**Claims**

1.    An aqueous formulation, comprisin:

a PEDF-derived short peptide (PDSP) having the sequence of SEQ ID NO: 1, 2, 3, 5, 6, 8. or 9,
boric acid at a concentration of 0.01 mM - 923 mM; and
a non-ionic tonicity agent.

2.    The aqueous formulation of claim 1, wherein the concentration of boric acid is from 10 mm - 100 mM.

3.    The aqueous formulation of claim 1, wherein the concentration of boric acid is from 20 mM - 50 mM.

4.    The aqueous formulation of claim 1, wherein a pH value is around 5.5 - 8.4.

5.    The aqueous formulation of claim 1, wherein a pH value is around 6.5 - 7.5.

6.    The aqueous formulation of claim 1, wherein the non-ionic tonicity agent is glycerin, sucrose, mannitol, or sorbitol.

7.    The aqueous formulation of claim 1, wherein the non-ionic tonicity agent is glycerin.

8.    The aqueous formulation of claim 7. wherein a concentration of glycerin is from 10 mM to 500 mM.

9.    The aqueous formulation of claim 7, wherein a concentration of glycerin is from 300 mM to 350 mM.

10.    The aqueous formulation of claim 1, wherein the non-ionic tonicity agent is sorbitol.

11.    The aqueous formulation of claim 10, wherein a concentration of sorbitol is from 100 mM to 400 mM.

12.    The aqueous formulation of claim 10, wherein a concentration of sorbitol is from 300 mM to 350 mM.

13.    The aqueous formulation of any one of claims 1-12, wherein the PDSP has the sequence of SEQ ID NO:3.

**14.** The aqueous formulation of claim 13. wherein a concentration of the PDSP is 0.01%-1% w/v.

**15.** The aqueous formulation of claim 13, wherein a concentration of the PDSP is 0.03% w/v.

**Patentansprüche**

**1.** Wässrige Formulierung, umfassend:

ein PEDF-abgeleitetes kurzes Peptid (PDSP) mit der Sequenz von SEQ ID NO: 1, 2, 3, 5, 6, 8 oder 9;
Borsäure in einer Konzentration von 0,01 mM bis 923 mM; und
ein nichtionisches Tonizitätsmittel.

**2.** Wässrige Formulierung nach Anspruch 1, wobei die Konzentration der Borsäure 10 mM bis 100 mM beträgt.

**3.** Wässrige Formulierung nach Anspruch 1, wobei die Konzentration der Borsäure zwischen 20 mM und 50 mM liegt.

**4.** Wässrige Formulierung nach Anspruch 1, wobei der pH-Wert etwa 5,5-8,4 beträgt.

**5.** Wässrige Formulierung nach Anspruch 1, wobei der pH-Wert etwa 6,5-7,5 beträgt.

**6.** Wässrige Formulierung nach Anspruch 1, wobei das nichtionische Tonizitätsmittel Glycerin, Saccharose, Mannitol oder Sorbitol ist.

**7.** Wässrige Formulierung nach Anspruch 1, wobei das nichtionische Tonizitätsmittel Glycerin ist.

**8.** Wässrige Formulierung nach Anspruch 7, wobei die Glycerinkonzentration zwischen 10 mM und 500 mM liegt.

**9.** Wässrige Formulierung nach Anspruch 7, wobei die Glycerinkonzentration zwischen 300 mM und 350 mM liegt.

**10.** Wässrige Formulierung nach Anspruch 1, wobei das nichtionische Tonizitätsmittel Sorbit ist.

**11.** Wässrige Formulierung nach Anspruch 10, wobei die Konzentration von Sorbit zwischen 100 mM und 400 mM liegt.

**12.** Wässrige Formulierung nach Anspruch 10, wobei die Konzentration von Sorbit zwischen 300 mM und 350 mM liegt.

**13.** Wässrige Formulierung nach einem der Ansprüche 1 bis 12, wobei das PDSP die Sequenz gemäß SEQ ID NO:3 aufweist.

**14.** Wässrige Formulierung nach Anspruch 13, wobei die Konzentration des PDSP 0,01 % bis 1 % w/v beträgt.

**15.** Wässrige Formulierung nach Anspruch 13, wobei die Konzentration des PDSP 0,03 % w/v beträgt.

**Revendications**

**1.** Formulation aqueuse, comprenant :

un peptide court dérivé du PEDF (PDSP) ayant la séquence de SEQ ID NO : 1, 2, 3, 5, 6, 8 ou 9;
de l'acide borique à une concentration de 0,01 mM à 923 mM ; et un agent tonique non ionique.

**2.** Formulation aqueuse selon la revendication 1, dans laquelle la concentration en acide borique est comprise entre 10 mM et 100 mM.

**3.** Formulation aqueuse selon la revendication 1, dans laquelle la concentration en acide borique est comprise entre 20 mM et 50 mM.

**4.** Formulation aqueuse selon la revendication 1, dans laquelle le pH est compris entre environ 5,5 et 8,4.

**5.** Formulation aqueuse selon la revendication 1, dans laquelle le pH est compris entre environ 6,5 et 7,5.

**6.** Formulation aqueuse selon la revendication 1, dans laquelle l'agent de tonicité non ionique est la glycérine, le saccharose, le mannitol ou le sorbitol.

**7.** Formulation aqueuse selon la revendication 1, dans laquelle l'agent de tonicité non ionique est la glycérine.

**8.** Formulation aqueuse selon la revendication 7, dans laquelle la concentration en glycérine est comprise entre 10 mM et 500 mM.

**9.** Formulation aqueuse selon la revendication 7, dans laquelle la concentration en glycérine est comprise entre 300 mM et 350 mM.

**10.** Formulation aqueuse selon la revendication 1, dans laquelle l'agent de tonicité non ionique est le sorbitol.

**11.** Formulation aqueuse selon la revendication 10, dans laquelle la concentration en sorbitol est comprise entre 100 mM et 400 mM.

**12.** Formulation aqueuse selon la revendication 10, dans laquelle la concentration en sorbitol est comprise entre 300 mM et 350 mM.

**13.** Formulation aqueuse selon l'une quelconque des revendications 1 à 12, dans laquelle le PDSP a la séquence SEQ ID NO:3.

**14.** Formulation aqueuse selon la revendication 13, dans laquelle la concentration en PDSP est comprise entre 0,01 % et 1 % p/v.

**15.** Formulation aqueuse selon la revendication 13, dans laquelle la concentration en PDSP est de 0,03 % p/v.

FIG. 1

**A.**

**10mM citrate buffer, pH6.0**

Original solution (0 hr)

0.85% NaCl | 334mM Glycerin

Precipitation

1.5hr | 4.5hr

Turbidity

2hr | 4.5hr

**B.**

**10mM citrate buffer, pH7.0**

Original solution (0 hr)

334mM Glycerin

Precipitation

5hr

Turbidity

7hr

**FIG. 2**

FIG. 3

FIG. 4

FIG. 5

FIG. 6

FIG. 7

**FIG. 8**

EP 4 281 099 B1

FIG. 9A

FIG. 9B

**EP 4 281 099 B1**

**REFERENCES CITED IN THE DESCRIPTION**

**Patent documents cited in the description**

- US 20100047212
- US 9884012 B
- US 9938328 B
- US 9777048 B
- US 9815878 B
- US 8507446 B